# EUROPEAN PATENT APPLICATION

(11) **EP 0 934 726 A1**
(43) Date of publication of application: **11.08.1999**
(21) Application number: 99300382.1
(22) Date of filing: 20.01.1999
(51) Int. Cl.: A61B 17/135

(54) **Inflatable collar**

(30) Priority: 07.02.1998 GB 9802588
(71) Applicant: Fisher, Peter, Doncaster, Yorkshire DN1 2LW (GB)
(72) Inventor: Fisher, Peter, Doncaster, Yorkshire DN1 2LW (GB)
(74) Representative: Kinton, Colin David

(57) **Abstract**

A device to restrict the flow of fluid through a flexible conduit, **characterised by:**
- at least one inflatable portion (3);
- adjustable means (7) to secure the device around the conduit;
- an inlet pipe (4) to admit an inflation medium to the inflatable portion (3);
- an outlet pipe (5) to release said inflation medium from the inflatable portion (3);
- **and further characterised in that** the outlet pipe (5) is adapted to be held, during use of the device, in a closed condition by closure means operatively associated with said outlet pipe (5) and causes at least partial deflation of said inflatable portion (3). In a preferred embodiment, the device provides means for safely restricting the flow of blood through a blood-vessel and means for the rapid release of such restriction. Thus, the device may be used as a tourniquet or to heighten the sensations experienced during sexual acts.

## Description

The present invention relates to an inflatable device adapted to exert pressure and to restrict the flow of fluid through a flexible conduit, the device having a quick-release valve operable (or self-operating) if the pressure becomes too great.

It is known in the art to use tourniquets to restrict the flow of blood to open wounds to prevent blood loss, bloodflow being restricted by tightly securing a strip of material around a portion of the body e.g. a limb containing the artery supplying the wound site. However, application of high pressure to a blood vessel for a long period of time can result in undesirable side-effects, such as gangrene or loss of consciousness.

It is also known to restrict bloodflow to the brain and respiratory system during sexual acts to heighten the sensation experienced. In general the bloodflow is restricted by means of a belt, a rope, the arm of another person or other suitable means. For example, the person may tie the rope or belt to a protrusion of a suitable height and exert pressure by means of the rope or belt by hanging themselves from the protrusion in such a manner that they can reach the floor when required and thereby release the pressure. However, as the air/bloodflow becomes restricted the person may begin to lose consciousness and therefore lose the ability to release the pressure, which can result in asphyxiation through strangulation.

It is therefore desirable to provide a means to release pressure, prior to or on the onset of loss of consciousness or of other undesirable side-effects.

It is an object of the present invention to provide an inflatable device for safely restricting the flow of fluid through a flexible conduit, the device being provided with means to enable rapid deflation when necessary, thus overcoming the problems associated with the prior art methods.

According to a first aspect, the present invention provides a device to restrict the flow of fluid through a flexible conduit, the device comprising at least one inflatable portion, adjustable securing means to secure the device around the conduit, an inlet pipe and an outlet pipe for an inflation medium, in which the outlet pipe is open in its normal position and is held closed during use by the operation of a closure means at an end portion thereof, whereby release of the closure means reopens the outlet and causes at least partial deflation of the inflatable portion.

The inflatable portion (which is preferably tubular), may be secured circumferentially to at least one arcuate portion of a strip of a resilient material such as leather, suede or polyvinyl chloride, such that the tube inflates in one radial direction only.

The resilient material may have an ornamental finish.

The tube is preferably secured to the resilient material by means of a suitable adhesive.

The adjustable securing means is preferably corresponding strips of a hook-pile material such as VELCRO (RTM) provided on inflatable portion or the resilient material (where such is provided) and positioned such that they contact each other when the device is wrapped around a flexible conduit.

Alternatively, the securing means may comprise a buckle, buttons, press-studs or other suitable fastening means.

The inlet pipe allows air to be pumped into the inflatable portion, e.g. by a hand pump or bellows. Alternatively, air can be supplied to the inflatable portion by a person blowing into the inlet pipe. The inlet pipe or pump/bellow is preferably provided with a non-return valve.

The outlet pipe is preferably held closed by pressure applied by the user's hand or fingers. The outlet pipe may be provided with an aperture and a closure means which can be held over the aperture by the user such that the closure means moves away from the aperture when the user releases pressure on it. The closure means may be provided with a spring to urge it out of engagement with the aperture when the pressure is released, or it may be adapted to disengage from the aperture if the pressure of air in the tube exceeds a predetermined valve.

The outlet pipe therefore acts as a "deadman's handle", that is to say as soon as pressure is removed from the outlet pipe or from any provided closure means the pipe or closure means opens to allow air from the inflatable portion to escape. This safety feature allows the inflatable tubing to be deflated quickly and easily. For example, if the user is manually maintaining the outlet closed then there will be an automatic release of the closure means if the user tends to lose consciousness.

According to a second aspect, the present invention provides a method of restricting fluid flow through a flexible conduit by means of a device according to the first aspect of the present invention, the method comprising the steps of wrapping the inflatable portion around a part of the body and securing it in position by using the adjustable securing means, closing the outlet pipe by applying pressure to it, and inflating the inflatable portion by way of the inlet pipe.

Preferably, the outlet pipe is held closed by means of the finger and thumb of a user.

The inflation of the device may be achieved by means of a bellows or other hand pump.

The device can be used as a tourniquet around the limbs of a body to restrict bloodflow when an accident or injury has occurred resulting in an open wound.

The device can also be used around the neck of a person during sexual acts to restrict bloodflow and breathing and increase the sensations felt.

In the event that the bloodflow and breathing is restricted too much the user may lose consciousness and accordingly the user's hand will fall away from the outlet pipe allowing the inflatable tube to deflate and bloodflow to be restored. When the device is being used as a tourniquet, the restoration of bloodflow will help to avoid gangrene at the site of the wound.

The device will now be illustrated by way of example only and with reference to the accompanying drawings in which:-
**Figure 1** shows a longitudinal cross section through the device; and
**Figure 2a + 2b** show two possible closure means for the outlet pipe.

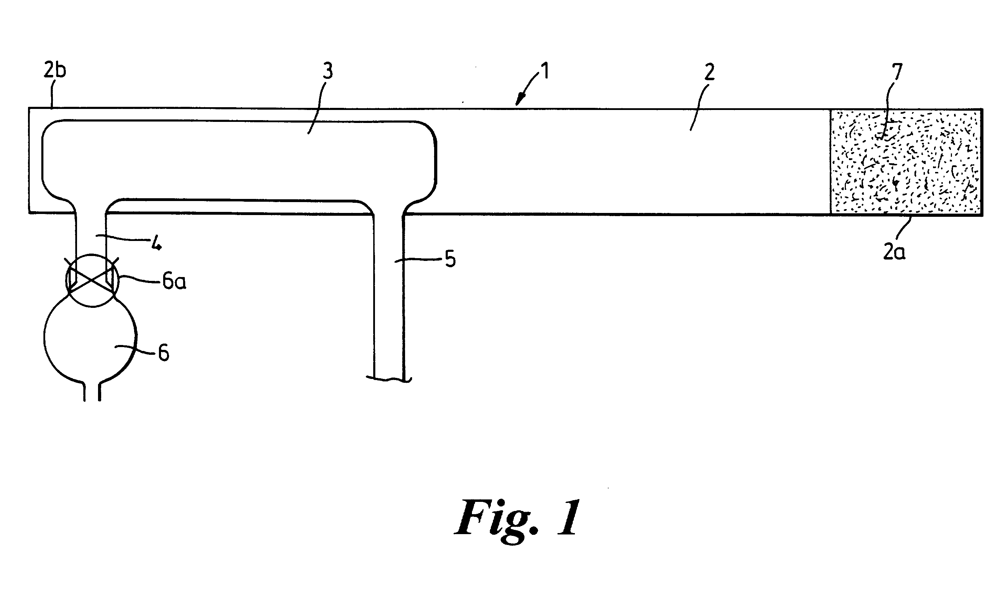

Figure 1 shows a device 1 to restrict flow of fluid through a flexible conduit. The device comprises a strip 2 of resilient material (such as suede) having secured thereto by a suitable adhesive a portion of inflatable tubing 3. The tubing 3 has an inlet pipe 4 and an outlet pipe 5 to allow the tube 3 to be inflated and deflated. The tube 3 is inflated using a hand held pump 6 fitted onto the inlet pipe 4. The pump 6 or outlet pipe 5 is provided with a suitable on way non-return valve shown schematically at 6a.

The strap of resilient material 2 has provided a portion 7 of VELCRO (RTM) on an end 2a in the inner side (shown in Figure 1) and a corresponding portion of VELCRO (RTM) is provided on the opposite end 2b of the strap, on the outer side (not shown).

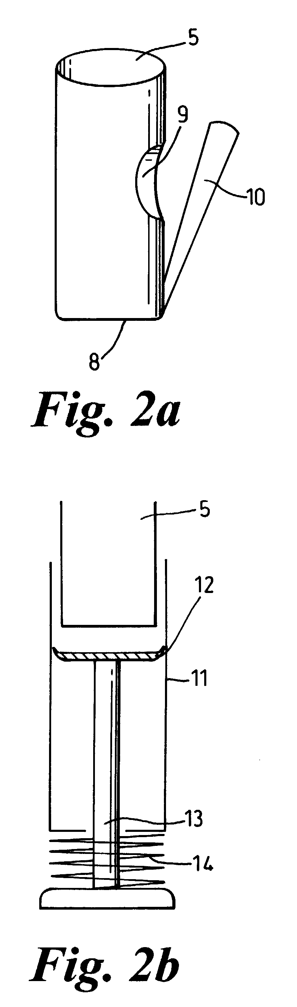

The outlet pipe 5 may be held closed during inflation and use of the device by squeezing the outlet pipe 5 between finger and thumb, Figures 2a and 2b show alternative arrangements for closing the outlet pipe 5.

Figure 2a shows a section of outlet pipe 5 provided with a sealed end 8 and an aperture 9 which can be closed by pressing the hinged cover means 10 against the aperture 9. The cover means 10 must be held against the aperture 9 by the user of the device in order to maintain the air pressure in the device.

Figure 2b shows a piece of rigid pipe 11 having a plunger 12 inside it. The handle 13 of plunger 12 protrudes from the pipe 11 and is urged outwardly of the pipe by a spring 14. The pipe 11 fits over the outlet pipe 5 and pressing the handle 13 forces the plunger 12 against the end of the outlet pipe 5, thus sealing it. If the handle 13 is released, the spring 14 urges the plunger out of engagement with the outlet pipe 5 and the pressure in the device is released.

In the devices shown in Figures 2a and 2b the outlet pipe 5 needs to be manufactured from a fairly rigid material such as high-density polyethylene to avoid deformation of the pipe on closure.

In use in a first embodiment, the device 1 is wrapped around a limb of a person who has an open bleeding wound on that limb and secured in position by the VELCRO (RTM) portion 7. The outlet pipe 5 is held closed while the portion 3 is inflated by means of the inlet pipe 4 and the hand pump 6 to restrict the bloodflow to the wound. The pressure applied to the limb can be reduced or released by allowing air to escape from the outlet pipe 5.

In a second embodiment, the device 1 is wrapped around the neck of a person during sexual acts to heighten the sensations experienced. The device 1 is secured in place by the VELCRO (RTM) portion 7 and inflated by means of the hand pump 6 and inlet pipe 4 while applying finger and thumb pressure to the outlet pipe 5 to prevent air escaping.

If the device 1 becomes too tight the pressure on the outlet pipe can be reduced to allow more air to escape. In the event that the bloodflow and breathing of the user is restricted for too long and the person loses consciousness, the outlet pipe 5 will be opened as the pressure on it is released by the hand falling away from the pipe 5. Thus the portion 3 of device 1 will deflate and bloodflow, breathing and therefore consciousness, will be restored.

## Claims

1. A device to restrict the flow of fluid through a flexible conduit, **characterised by:**
- at least one inflatable portion (3);
- adjustable securing means (7) to secure the device around the conduit;
- an inlet pipe (4) to admit an inflation medium to the inflatable portion (3);
- **and further characterised in that** the outlet pipe (5) is adapted to be held, during use of the device, in a closed condition by closure means operatively associated with said outlet pipe (5), whereby release of said closure means re-opens said outlet pipe (5) and causes at least partial deflation of said inflatable portion (3).

2. A device according to Claim 1, **characterised in that** the inflatable portion (3) is of substantially tubular configuration.

3. A device according to Claim 2, **characterised in that** the inflatable portion (3) is secured circumferentially to at least one arcuate portion of a strip (2) of a resilient material, whereby inflation of the inflatable portion (3) takes place in one radial direction only.

4. A device according to Claim 3, **characterised in that** the strip (2) is made of leather, suede or polyvinyl chloride.

5. A device according to Claim 3 or Claim 4, **characterised in that** the strip (2) is provided with an ornamental finish.

6. A device according to any one of Claims 1 to 5, **characterised in that** the adjustable securing means (7) comprises complementary portions of a hook-pile material.

7. A device according to any one of Claims 1 to 5, **characterised in that** the adjustable securing means (7) comprises one or more buckles, buttons or press-studs.

8. A device according to any one of Claims 1 to 7, **characterised in that** the inflation medium is supplied to the inlet pipe (4) by means of a pump (6).

9. A device according to Claim 8, **characterised in that** the inlet pipe (4) is provided with a one-way valve (6a).

10. A device according to any one of Claims 1 to 9, **characterised in that** the inflation medium is air.

11. A device according to any one of Claims 1 to 10, **characterised in that** the closure means for the outlet pipe (5) comprises manual pressure exerted on the pipe (5) by the user.

12. A device according to any one of Claims 1 to 10, **characterised in that** the closure means for the outlet pipe (5) comprises a sealed end (8) and an aperture (9) closable by means of a hinged cover (10).

13. A device according to any one of Claims 1 to 10, **characterised in that** the closure means for the outlet pipe (5) comprises a rigid pipe (11) containing a plunger (12) operatively associated with a handle (13) and a spring (14), the rigid pipe (11) being adapted to fit over and sealingly engage the outlet pipe (5).

14. The use of a device according to any one of Claims 1 to 13 as a tourniquet.

15. The use of a device according to any one of Claims 1 to 13 to heighten the sensations experienced during sexual acts.
